# EUROPEAN PATENT SPECIFICATION

(11) **EP 1 339 723 B1**
(45) Date of publication and mention of the grant of the patent: **08.03.2006**
(21) Application number: 01999226.2
(22) Date of filing: 19.11.2001
(51) Int. Cl.: C07D 501/08, C07D 501/18, C07D 501/20, C07D 405/12, C07F 9/568, C07D 205/095, C07D 513/04, C07D 277/00, C07D 205/00

(54) **PROCESS AND ESTER DERIVATIVES USEFUL FOR PREPARATION OF CEPHALOSPORINS**
VERFAHREN UND ESTERDERIVATE ZUR HERSTELLUNG VON CEPHALOSPHORINEN
PROCEDE ET DERIVES D'ESTER UTILES POUR LA PREPARATION DE CEPHALOSPORINES

(30) Priority: 04.12.2000 US 251018 P
(43) Date of publication of application: 03.09.2003
(73) Proprietor: Pfizer Products Inc., Groton, Connecticut 06340 (US)
(72) Inventor: COLBERG, J. C., Pfizer Global Res. and Developmt., Groton, CT 06340 (US); TUCKER, J. L., Pfizer Global Res. and Devlpomt., San Diego, CA 92121 (US); ZENONI, M., ACS Dobfar S.p.A., I-20067 Tribiano (IT); FOGLIATO, Giovanni, ACS Dobfar S.p.A., I-20067 Tribiano (IT); DONADELLI, Alessandro, ACS Dobfar S.p.A., I-20067 Tribiano (IT); NAGAKURA, Isao, Tochigi 326-0824 (JP); MORITA, Hiromasa, Aichi 478-0003 (JP); MATSUO, Hideyuki, Nishimatsuura-gun, Saga 844-0012 (JP)
(74) Representative: Lane, Graham Mark Hamilton
(86) International application number: PCT/IB2001/002181
(87) International publication number: WO 2002/046199

(56) References cited:
- WO-A-92/01695
- WO-A-92/01696
- WO-A-93/25551
- WO-A-96/17847
- GB-A- 2 300 856

## Description

### BACKGROUND OF THE INVENTION

The invention relates to novel processes for the preparation of para-nitrobenzyl esters and allyl esters useful in the preparation of 3-cyclic-ether-substituted cephalosporins. The invention also relates to novel processes for preparing the above para-nitrobenzyl esters and allyl esters by the use of trimethylphosphine. The invention also relates to 3-cyclic-ether-substituted cephalosporins. These compounds possess certain advantageous properties, such as crystalline form and high enantiomeric excess (e.e.).

The 3-cyclic-ether-substituted cephalosporins prepared by the methods of the present invention have prolonged and high levels of antibacterial activity and possess good absorption parentally in humans and animals. The 3-cyclic-ether-substituted cepha.osporins prepared by the processes of the present invention contain a cyclic ether substituent at the 3-position of the cephalosporin nucleus.

GB 1405758 describes alternative methods of preparation of certain 3-cyclic-ether-substituted cephalosporins.

J. Antibiotics (1994), vol. 47(2), page 253, and WO 92/01696 also describe alternative methods of preparation of compounds of formula (I), as defined herein below, and compounds useful in said processes.

United States Patents No. 6,020,329 and 6,077,952 describe salts, polymorphs, solvates ard hydrates of 3-cyclic-ether-substituted cephalosporins.

United States Patent No. 6,001,997 describes alternative preparations of 3-cyclic-ether-substituted cephalosporins.

United States Provisional Patent Application entitled "Coupling Process And Intermediates Useful For Preparing Cephalosporins", filed November 30, 2000, refers to intermediates and processes to prepare 3-cyclic-ether-substituted cephalosporins.

Each of the above referenced publications, patents and patent applications is hereby incorporated by reference in its entirety.

WO 96/17847 describes alternative methods of preparation of certain 3-cyclic-ether substituted cephalosporins.

WO 93/25551 describes certain carbacephem compounds bearing a cyclic ether or thio-ether substituent at the 3-position of the cephem nucleus.

The present inventors have discovered a novel compound of formula (IIIa), as defined herein below, useful for the preparation of compounds of formula (I), as defined herein below. The present inventors have also discovered a high-yielding process for the preparation of said compounds of formula (I).

### Summary of the Invention

The present invention relates to a process for preparing a compound of formula **(I)** 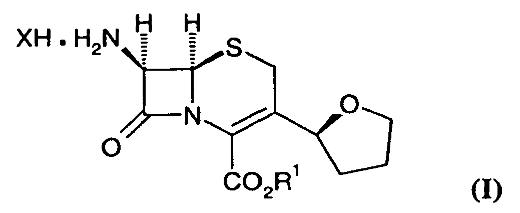
wherein R¹ is para-nitrobenzyl or allyl; preferably para-nitrobenzyl; X is halo selected from the group consisting of bromo, chloro, fluoro and iodo, preferably chloro; by:
a) reacting a compound of formula **(IIIc):** 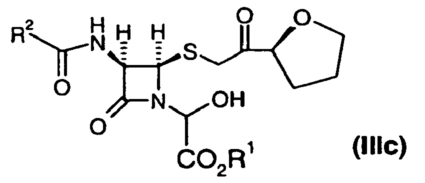
   wherein R¹ is *para*-nitrobenzyl or allyl, preferably para-nitrobenzyl; and R² is selected from the group consisting of C₁₋₆alkyl, C₆₋₁₀aryl, C₆₋₁₀arylC₁₋₆alkyl and dithianyl, preferably C₆₋₁₀arylC₁₋₆alkyl, such as benzyl; with a halogenating agent, in a solvent and in the presence of a base;
b) reacting the resulting compound of formula **(IIIb)** 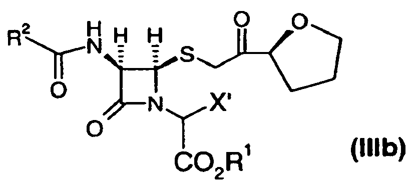
   wherein R¹ is para-nitrobenzyl or allyl, preferably *para*-nitrobenzyl; R² is selected from the group consisting of C₁₋₆alkyl, C₆₋₁₀aryl, C₆₋₁₀arylC₁₋₆alkyl and dithianyl; preferably
   C₆₋₁₀arylC₁₋₆alkyl, such as benzyl; and X' is halo, preferably chloro; with trimethylphosphine, in a solvent and in the presence of a base;
c) cyclizing the resulting trimethylphosphinic compound of formula **(IIIa):** 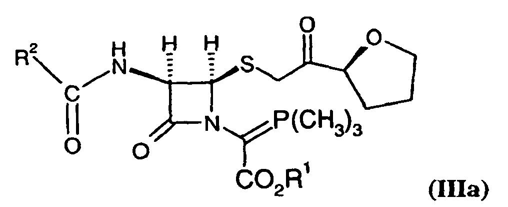
   wherein R¹ is para-nitrobenzyl or allyl, preferably para-nitrobenzyl; and R² is selected from the group consisting of C₁₋₆alkyl, C₆₋₁₀aryl C₆₋₁₀arylC₁₋₆alkyl and dithianyl, preferably C₆₋₁₀arylC₁₋₆alkyl, such as benzyl; in a solvent; and
d) reacting the resulting compound of formula (II) 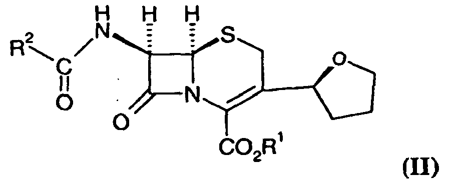
   wherein R¹ is para-nitrobenzyl or allyl, preferably para-nitrobenzyl; and
   R² is selected from the group consisting of C₁₋₆alkyl, C₆₋₁₀aryl, C₆₋₁₀arylC₁₋₆alkyl and dithianyl; preferably C₆₋₁₀arylC₁₋₆alkyl, such as benzyl;
   with an acid to form said compound of formula **(I)**.

The term "alkyl", as used herein, unless otherwise indicated, includes saturated monovalent hydrocarbon radicals having straight, branched moieties or combinations thereof. Alkyl groups, wherever they occur, may be optionally substituted by a suitable substituent.

The term "cycloalkyl", as used herein, unless otherwise indicated, includes a mono or bicyclic carbocyclic ring (e.g., cyclopropyl, cyclobutyl, cyclopentyl, cyclohexyl, cycloheptyl, cyclooctyl, cyclononyl, cyclopentenyl, cyclohexenyl, bicyclo[2.2.1]heptanyl, bicyclo[3.2.1]octanyl and bicyclo[5.2.0]nonanyl, etc.); optionally containing 1 or 2 double bonds and optionally substituted by 1 to 3 suitable substituents as defined below such as fluoro, chloro, trifluoromethyl, (C₁₋₄)alkoxy, (C₆₋₁₀)aryloxy, trifluoromethoxy, difluoromethoxy or (C₁₋₄)alkyl, more preferably fluoro, chloro, methyl, ethyl and methoxy.

The term "alkoxy", as used herein, includes O-alkyl groups wherein "alkyl" is as defined above.

The term "halo", as used herein, unless otherwise indicated, includes fluorine, chlorine, bromine or iodine, preferably bromine or chlorine.

The term "aryl", as used herein, unless otherwise indicated, includes an organic radical derived from an aromatic hydrocarbon by removal of one or more hydrogen(s), such as phenyl or naphthyl, optionally substituted by 1 to 3 suitable substituents such as fluoro, chloro, cyano, nitro, trifluoromethyl, (C₁₋₆)alkoxy, (C₆₋₁₀)aryloxy, (C₃₋₈)cycloalkyloxy, trifluoromethoxy, difluoromethoxy, or (C₁₋₆)alkyl.

The term "heteroaryl", as used herein, unless otherwise indicated, includes an organic radical derived from an aromatic heterocyclic compound by removal of one or more hydrogen(s), such as benzimidazolyl, benzofuranyl, benzofurazanyl, 2H-1-benzopyranyl, benzothiadiazine, benzothiazinyl, benzothiazolyl, benzothiophenyl, benzoxazolyl, chromanyl, cinnolinyl, furazanyl, furopyridinyl, furyl, imidazolyl, indazolyl, indolinyl, indolizinyl, indolyl, 3H-indolyl, isoindolyl, isoquinolinyl, isothiazolyl, isoxazolyl, naphthyridinyl, oxadiazolyl, oxazolyl, phthalazinyl, pteridinyl, purinyl, pyrazinyl, pyridazinyl, pyridinyl, pyrimidinyl, pyrazolyl, pyrrolyl, quinazolinyl; quinolinyl, quinoxalinyl, tetrazolyl, thiazolyl, thiadiazolyl, thienyl, triazinyl and triazolyl, wherein said (C₁₋₁₀)heteroaryl is optionally substituted on any of the ring carbon atoms capable of forming an additional bond by one or two substituents independently selected from F, Cl, Br, CN, OH, (C₁₋₄)alkyl, (C₁₋₄)perfluoroalkyl, (C₁₋₄)perfluoroalkoxy, (C₁₋₄)alkoxy and (C₃₋₈)cycloalkyloxy. The foregoing groups, as derived from the compounds listed above, may be C-attached or N-attached where such is possible. For instance, a group derived from pyrrole may be pyrrol-1-yl (N-attached) or pyrrol-3-yl (C-attached).

The term "heterocyclyl", as used herein, unless otherwise indicated, includes an organic radical derived from a non-aromatic heterocyclic compound by removal of one or more hydrogen(s), such as 3-azabicyclo[3.1.0]hexanyl, 3-azabicyclo[4.1.0]-heptanyl, azetidinyl, dihydrofuranyl, dihydropyranyl, dihydrothienyl, dioxanyl, 1,3-dioxolanyl, 1,4-dithianyl, hexahydroazepinyl, hexahydropyrimidine, imidazolidinyl, imidazolinyl, isoxazolidinyl, morpholinyl, oxazolidinyl, piperazinyl, piperidinyl, 2H-pyranyl, 4H-pyranyl, pyrazolidinyl, pyrazolinyl, pyrrolidinyl, 2-pyrrolinyl, 3-pyrrolinyl, quinolizinyl, tetrahydrofuranyl, tetrahydropyranyl, 1,2,3,6-tetrahydropyridinyl, tetrahydrothienyl, tetrahydrothiopyranyl, thiomorpholinyl, thioxanyl and trithianyl. The foregoing groups, as derived from the compounds listed above, may be C-attached or N-attached where such is possible. For example, a group derived from piperidine may be piperidin-1-yl (N-attached) or piperidin-4-yl (C-attached). The foregoing groups, as derived from the compounds listed above, may be optionally substituted where such is possible by a suitable substituent, such as oxo, F, Cl, Br, CN, OH, (C₁₋₄)alkyl, (C₁₋₄)perfluoroalkyl, (C₁₋₄)perfluoroalkoxy, (C₁₋₄)alkoxy or (C₃₋₈)cycloalkyloxy.

The phrase "a suitable substituent" is intended to mean a chemically and pharmaceutically acceptable functional group *i*.*e*., a moiety that does not negate the inhibitory activity of the inventive compounds. Such suitable substituents may be routinely selected by those skilled in the art. Illustrative examples of suitable substituents include, but are not limited to halo groups, perfluoroalkyl groups, perfluoroalkoxy groups, alkyl groups, hydroxy groups, oxo groups, mercapto groups, alkylthio groups, alkoxy groups, aryl or heteroaryl groups, aryloxy or heteroaryloxy groups, aralkyl or heteroaralkyl groups, aralkoxy or heteroaralkoxy groups, carboxy groups, amino groups, alkyl- and dialkylamino groups, carbamoyl groups, alkylcarbonyl groups, alkoxycarbonyl groups, alkylaminocarbonyl groups dialkylamino carbonyl groups, arylcarbonyl groups, aryloxycarbonyl groups, alkylsulfonyl groups, arylsulfonyl groups and the like.

The term 'salts" is intended to mean the pharmaceutically acceptable acid or base addition salts of compounds of the formula (I).

The acids which are used to prepare the pharmaceutically acceptable acid addition salts of the aforementioned base compounds of this invention are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, *para-*toluenesulfonate and pamoate [i.e., 1,1'-methylene-bis-(2-hydroxy-3- naphthoate)]salts.

The bases that may be used as reagents to prepare pharmaceutically acceptable base salts of those compounds of formula (I) that are acidic in nature are those that form non-toxic base salts with such compounds. Such non-toxic base salts include, but are not limited to those derived from such pharmacologically acceptable cations such as alkali metal cations (e.g., potassium and sodium) and alkaline earth metal cations (e.g., calcium and magnesium), ammonium or water-soluble amine addition salts such as N-methylglucamine (meglumine), and the lower alkanolammonium and other base salts of pharmaceutically acceptable organic amines.

Some compounds of formula (I) contain chiral centers and therefore exist in different enantiomeric forms. This invention relates to all optical isomers, enantiomers, diastereomers and stereoisomers of the compounds of formula I and mixtures thereof. The compounds of the invention also exist in different tautomeric forms. This invention relates to all tautomers of formula (I). Those skilled in the art are well aware that the cephalosporin nucleus exists as a mixture of tautomers in solution. The various ratios of the tautomers in solid and liquid form is dependent on the various substituents on the molecule as well as the particular crystallization technique used to isolate a compound.

In one embodiment of the process of the invention for the conversion of compounds of formula (IIIa) into compounds of formula (II), said R¹ is allyl.

In another embodiment of the invention of the aforesaid conversion, said R² is C₁₋₆alkyl, such as methyl or ethyl. In another embodiment, said R² is C₆₋₁₀aryl, such as phenyl. In yet another embodiment, said R² is C₆₋₁₀arylC₁₋₆alkyl.

In a preferred embodiment of the aforesaid conversion, R¹, wherever it occurs, is para-nitrobenzyl; and R², wherever it occurs, is benzyl.

Suitable solvents for the aforesaid conversion include toluene, xylene, tetrahydrofuran, methylene chloride or acetonitrile. Preferably the solvent is methylene chloride.

The aforesaid conversion of compounds of formula **(IIIa)** into compounds of formula **(II)** may be conducted at a temperature of from about 40°C to about 160°C; preferably about 65°C. The aforesaid conversion may be conducted for a period from about 1 hour to about 24 hours, preferably about 16 hours.

In a preferred embodiment of the aforesaid step d) of the process of the invention, R¹, wherever it occurs, is para-nitrobenzyl; and R², wherever it occurs, is benzyl.

Suitable acids in said process of the invention for the conversion of compounds of formula **(II)** into compounds of formula **(I)** include Lewis Acids, such as phosphorus pentachloride or phosphorus pentabromide; preferably phosphorus pentachloride.

Said process of the invention for the conversion of compounds of formula **(II)** into compounds of formula **(I)** is conducted at a temperature of from about -40°C to about +40°C: preferably from about -40°C to about +30°C. The aforesaid conversion may be conducted for a period of from about 1 hour to about 24 hours, preferably about 1 hour.

Suitable solvents for the aforesaid conversion include toluene, xylene, tetrahydrofuran, methylene chloride or acetonitrile. Preferably the solvent is methylene chloride.

Suitable solvents for the conversion of compounds of formula **(IIIb)** into compounds of formula **(IIIa)** include tetrahydrofuran, acetonitrile methylene chloride or mixtures thereof; preferably tetrahydrofuran.

Suitable bases for work up include imidazole, 2,6-lutidine, pyridine, N-methylmorpholine or sodium bicarbonate. In one embodiment of the invention, the base is 2,6-lutidine or N-methylmorpholine. In another embodiment of the invention, the base is pyridine. In a preferred embodiment of the invention, the base is sodium bicarbonate. The aforesaid conversion is conducted in with the suitable base during work up.

Said process of the invention for the aforesaid conversion of compounds of formula **(IIIb)** into compounds of formula **(IIIa)** may be conducted at a temperature of from about - 40°C to about -20°C; preferably of from about -40°C. The aforesaid conversion may be conducted for a period of from about 30 minutes to about 1 hour, preferably about 1 hour.

Suitable halogenating agents of the aforesaid process for conversion of compounds of formula **(IIIc)** into compounds of formula **(IIIb)** of the invention include thionyl chloride, thionyl bromide, phosphorus trichloride or phosphorus tribromide. Preferably, the halogenating agent is thionyl chloride.

Suitable solvents of the aforesaid conversion of the invention include methylene chloride or tetrahydrofuran. Preferably, the solvent is methylene chloride.

Suitable bases of the aforesaid conversion of the invention include pyridine, 2,6-lutidine, N-methylmorpholine or imidazole. In one embodiment of the invention, the base is 2,6-lutidine or N-methylmorpholine. In another embodiment of the invention, the base is pyridine. In another embodiment of the invention, the base is imidazole. In a preferred embodiment, the base is 2,6-lutidine.

Said process of the invention for the aforesaid conversion is conducted at a temperature of from about -40°C to about -20°C, preferably about -20°C. The aforesaid conversion may be conducted for a period of from about 15 minutes to about 1 hour, preferably about 1 hour.

Compounds of formula **(IIIc),** as defined above, may be prepared by reacting a compound of formula **(V)** 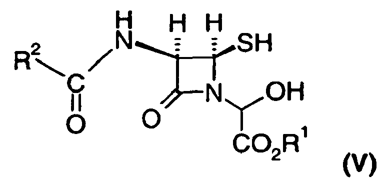
wherein R¹ is para-nitrobenzyl or allyl, preferably para-nitrobenzyl; and R² is selected from the group consisting of C₁₋₆alkyl, C₆₋₁₀aryl, C₆₋₁₀arylC₁₋₆alkyl and dithianyl, preferably C₆₋₁₀arylC₁₋₆alkyl, such as benzyl; with a compound of formula **(IV)** 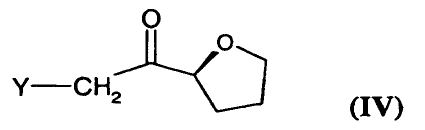
wherein Y is a leaving group; in the presence of a solvent, optionally in the presence of a base.

Suitable leaving groups of the aforesaid compound of formula **(IV)** include bromo, chloro, fluoro, iodo and tosylate, preferably bromo or chloro, most preferably bromo.

Suitable solvents for the aforesaid process for the conversion of compounds of formula **(V)** into compounds of formula **(IIIc)** include alcohols selected from the group consisting of methanol, ethanol and propanol; methylene chloride; acetone; dimethylformamide or mixtures thereof. In another embodiment, the solvent is methylene chloride. In another embodiment, the solvent is a mixture of acetone and alcohol, such as methanol. Preferably the solvent is acetone.

Said process for the conversion of compounds of formula **(V)** into compounds of formula **(IIIc)** may be conducted at a temperature of from about 10°C to about 25°C. preferably about 20 °C. The aforesaid conversion may be conducted for a period of from about 2 hours to about 24 hours, preferably about 4 hours.

In one embodiment of the aforesaid conversion, the reaction is performed in the presence of base, such as isopropylamine, pyridine or potassium carbonate; preferably pyridine. Preferably the aforesaid conversion is conducted without a base.

In another embodiment of the aforesaid process for the conversion of compounds of formula **(V)** into compounds of formula **(IIIc),** the compound of formula **(IV)** may be prepared *in situ,* by reacting the compound of formula **(V)** with a compound of formula **(IVa)** 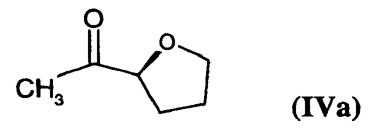
with an aqueous or an alcoholic solution of bromine, chlorine or iodine; and exposing the aqueous or alcoholic solution to an acid. Suitable acids include para-toluene sulfonic acid, perchloric acid or diluted phosphoric acid; preferably para-toluene sulfonic acid. In said *in situ* preparation, the preferred solvent is alcohol, such as methanol. The aforesaid preparation may be conducted for 2 hours at 60°C.

Compounds of formula **(V)** may also be prepared by reacting a compound of formula **(VIa)** 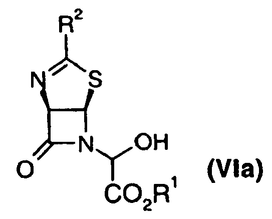
wherein R¹ is para-nitrobenzyl or allyl, preferably para-nitrobenzyl; and wherein R² is selected from the group consisting of C₁₋₆alkyl, C₆₋₁₀aryl, C₆₋₁₀arylC₁₋₆alkyl and dithianyl, preferably C₆₋₁₀arylC₁₋₆alkyl, such as benzyl; with an acid in a solvent.

Said process for the aforesaid conversion of compounds of formula **(VIa)** into compounds of formula **(V)** is conducted at a temperature of from about 20°C to about 25°C, preferably about 20°C. The aforesaid conversion may be conducted for a period of from about 2 hours to about 24 hours, preferably about 2 hours.

Suitable acids of the aforesaid process include para-toluene sulfonic acid or methane sulfonic acid. The preferred acid is para-toluene sulfonic acid.

Suitable solvents of the aforesaid process include methylene chloride, tetrahydrofuran, acetone or mixtures thereof. Preferably, the solvent is acetone.

Compounds of formula **(VIa)** may also be prepared by reacting a compound of formula **(VIb)** 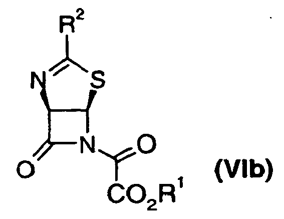
wherein R¹ is para-nitrobenzyl or allyl, preferably para-nitrobenzyl; and wherein R² is selected from the group consisting of C₁₋₆alkyl, C₆₋₁₀aryl, C₆₋₁₀arylC₁₋₆alkyl and dithianyl, preferably C₆₋₁₀arylC₁₋₆alkyl, such as benzyl; with a reducing agent, in a solvent.

Suitable reducing agents for the aforesaid process for the aforesaid conversion of compounds of formula **(VIb)** into compounds of formula **(VIa)** include sodium borohydride, sodium cyanoborohydride, borane or sodium triacetoxy borohydride. In one embodiment, the reducing agent is sodium borohydride. Preferably, the reducing agent is sodium triacetoxyborohydride or sodium borohydride. Most preferably, the reducing agent is sodium triacetoxyborohydride.

Suitable solvents for the aforesaid conversion include acetic acid, methylene chloride, tetrahydrofuran or mixtures thereof. Preferably the solvent is methylene chloride. When the reducing agent is sodium borohydride, the preferred solvent is acetic acid.

The aforesaid conversion may be conducted at a temperature of from about 20°C to about 66°C. The aforesaid conversion may be conducted for a period of from about 4 hours to about 24 hours.

Compounds of formula **(VIa)** may also be prepared by reacting a compound of formula **(XI)** 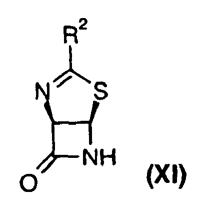
wherein R² is selected from the group consisting of C₁₋₆alkyl, C₆₋₁₀aryl, C₆₋₁₀arylC₁₋₆alkyl and dithianyl; preferably C₆₋₁₀arylC₁₋₆alkyl, such as benzyl; with a compound of formula **(X)** 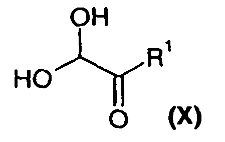
wherein R¹ is para-nitrobenzyl or allyl, preferably *para*-nitrobenzyl; in a solvent; in the presence of a base, preferably a catalytic amount of base.

Suitable solvents for the aforesaid process of the invention for the conversion of compounds of formula (XI) into compounds of formula **(VIa)** include methylene chloride, tetrahydrofuran or mixtures thereof. In one embodiment of the invention, the solvent is 1:1 mixture of methylene chloride and tetrahydrofuran. Preferably, the solvent is methylene chloride.

Suitable bases of the aforesaid conversion include diisopropylamine, triethylamine, pyridine or 2,6-lutidine. Preferably, the base is triethylamine. More preferably, the base is catalytic triethylamine.

The aforesaid conversion may be conducted at a temperature of from about 20°C to about 25°C. The aforesaid conversion may be conducted for a period of from about 30 minutes to about 2 hours, preferably about 1 hour.

Compounds of formula **(VIb)** may also be prepared by reacting a compound of formula **(VIII)** 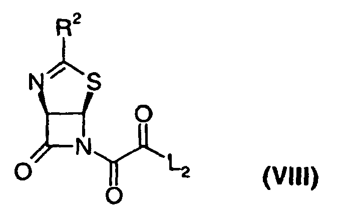
wherein R² is selected from the group consisting of C₁₋₆alkyl, C₆₋₁₀aryl, C₆₋₁₀arylC₁₋₆alkyl and dithianyl; preferably C₆₋₁₀arylC₁₋₆alkyl, such as benzyl; and L₂ is a leaving group; with a compound of formula **(VII)**

R¹―OH (VII)

wherein R¹ is para-nitrobenzyl or allyl, preferably para-nitrobenzyl, in a solvent, in the presence of a base.

Suitable L₂ leaving groups of the compound of formula **(VII)** include halo, azide or C₁₋₆alkoxy; preferably halo, such as chloro or bromo.

Suitable solvents of the aforesaid conversion of compounds of formula **(VIII)** into compounds of formula **(VIb)** include methylene chloride, tetrahydrofuran or mixtures thereof; preferably methylene chloride.

Suitable bases of the aforesaid conversion include diisopropylamine, triethylamine, pyridine and 2,6-lutidine; preferably triethylamine.

The aforesaid conversion may be conducted at a temperature of from about -78°C to about 25°C, preferably about -78°C. The aforesaid conversion may be conducted for a period of from about 5 minutes to about 10 minutes, preferably about 5 minutes.

In the aforesaid process for the conversion of compounds of formula **(VIII)** into compounds of formula **(VIb),** compounds of formula **(VIII)** can be prepared by reacting a compound of formula **(XI)** 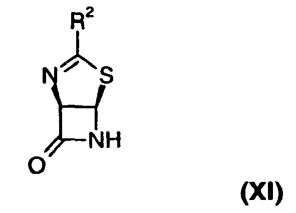
wherein R² is selected from the group consisting of C₁₋₆alkyl, C₆₋₁₀aryl, C₆₋₁₀arylC₁₋₆alkyl and dithianyl; preferably C₆₋₁₀arylC₁₋₆alkyl, such as benzyl; with a compound of formula **(IX)** 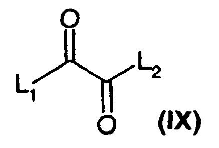
wherein each of said L₁ and L₂ is a leaving group, in a solvent, in the presence of a base.

Suitable L₁ and L₂ leaving groups of the compound of formula **(IX)** include halo, azide and C₁₋₆alkoxy; preferably halo, such as bromo and chloro.

Suitable solvents for the aforesaid process for the conversion of compounds of formula **(XI)** into compounds of formula **(VIII)** include methylene chloride, tetrahydrofuran or mixtures thereof; preferably methylene chloride.

Suitable bases of the aforesaid process include diisopropylamine, triethylamine, pyridine and 2,6-lutidine; preferably triethylamine.

Said aforesaid process is conducted at a temperature of from about -78°C to about 25°C, preferably about -78°C. The aforesaid conversion may be conducted for a period of from about 5 minutes to about 10 minutes, preferably about 5 minutes.

In the aforesaid conversion of compounds of formula **(XI)** into compounds of formula **(VIII),** said compounds of formula **(VIII)** may be isolated or they may be carried on directly to form compounds of formula **(VIb)** in a one pot reaction, as described above. Preferably, compounds of formula **(VIII)** are isolated before being converted to compounds of formula **(VIb).**

Compounds of formula **(VIb)** may also be prepared by reacting a compound of formula **(VIc)** 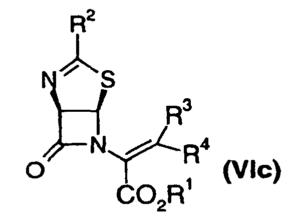
wherein R¹ is para-nitrobenzyl or allyl; R² is selected from the group consisting of C₁₋₆alkyl, C₆₋₁₀aryl, C₆₋₁₀arylC₁₋₆alkyl and dithianyl; preferably C₆₋₁₀arylC₁₋₆alkyl, such as benzyl; R³ is hydrogen or C₁₋₆alkyl; preferably C₁₋₆alkyl, such as methyl; and R⁴ is hydrogen or C₁₋₆alkyl; preferably C₁₋₆alkyl, such as methyl; with an oxidizing agent, in a solvent.

Suitable oxidizing agents for the aforesaid conversion of compounds of formula **(VIc)** into compounds of formula **(VIb)** include ozone.

Suitable solvents of the aforesaid conversion include methylene chloride, tetrahydrofuran, alcohol (such as isopropanol) or mixtures thereof. Preferably the solvent is a mixture of methylene chloride and isopropanol.

The aforesaid conversion may be conducted at a temperature of -70°C. The aforesaid conversion may be conducted for a period of from about 1 hour to about 24 hours, preferably about 6 hours.

Compounds of formula **(VIb)**, as defined above, may also be prepared by reacting a compound of formula **(XI)** 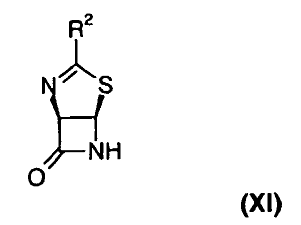
wherein R² is selected from the group consisting of C₁₋₆alkyl, C₆₋₁₀aryl, C₆₋₁₀aryl C₁₋₆alkyl, and dithianyl; preferably C₆₋₁₀arylC₁₋₆alkyl, such as benzyl; with a compound of formula **(XII)** 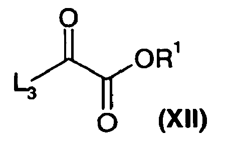
wherein L₃ is halo, such as chloro or bromo, and R¹ is para-nitrobenzyl or allyl; preferably para-nitrobenzyl, in a solvent, in the presence of a base.

Suitable solvents for the aforesaid process for the conversion of compounds of formula **(XI)** into compounds of formula **(VIb)** include methylene chloride, tetrahydrofuran or mixtures thereof; preferably methylene chloride.

Suitable bases of the aforesaid conversion include diisopropylamine, triethylamine, pyridine or 2,6-lutidine. Preferably, the base is triethylamine.

Said conversion may be conducted at a temperature of from about -40°C to about 25°C; preferably from about 20°C to about 25°C. The aforesaid conversion may be conducted for a period of from about 5 minutes to about 15 minutes, preferably about 10 minutes.

The present invention also relates to a compound of formula **(I)** 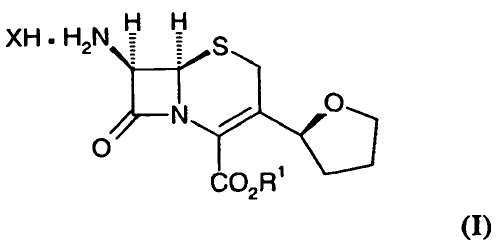
wherein R¹ is *para*-nitrobenzyl or allyl; and X is halo.

The compounds of formula **(I)** is useful in the high-yielding preparation of 3-cyclic-ether-substituted cephalosporins. These compounds possess certain advantageous properties, such as crystalline form and high enantiomeric excess (e.e.).

In one embodiment of the compound of formula **(I)** of the invention, R¹ is allyl. In another embodiment of the invention, R¹ is allyl and X is halo such as chloro or bromo, preferably chloro.

In a preferred embodiment of the compound of formula **(I)** of the invention, R¹ is *para-*nitrobenzyl. In a more preferred embodiment of the invention, R¹ is para-nitrobenzyl and X is chloro.

Specific compounds of the invention include Compounds of formula **(I)** including:
7-Amino-8-oxo-3-(tetrahydrofuran-2-yl)-5-thia-1-aza-bicyclo[4.2.0]oct-2-ene-2-carboxylic acid 4-nitro-benzyl ester;
7-Amino-8-oxo-3-(tetrahydrofuran-2-yl)-5-thia-1-aza-bicyclo[4.2.0]oct-2-ene-2-carboxylic acid allyl ester;

and salts thereof.

In addition, Compounds of formula **(II)** include:
8-Oxo-7-phenylacetylamino-3-(tetrahydrofuran-2-yl)-5-thia-1-aza-bicyclo[4.2.0]oct-2-ene-2-carboxylic acid 4-nitro-benzyl ester;
8-Oxo-7-phenylacetylamino-3-(tetrahydrofuran-2-yl)-5-thia-1-aza-bicyclo[4,2.0]oct-2-ene-2-carboxylic acid allyl ester;

and salts thereof.

Compounds of formula **(III)** include:
{2-Oxo-4-[2-oxo-2-(tetrahydrofuran-2-yl)-ethylsulfanyl)-3-phenylacetylamino-azetidin-1-yl}-(trimethyl-α-phosphanylidene)-acetic acid 4-nitro-benzyl ester;
{2-Oxo-4-[2-oxo-2-(tetrahydrofuran-2-yl)-ethylsulfanyl]-3-phenylacetylamino-azetidin-1-yl}-(trimethyl-α-phosphanylidene)-acetic acid allyl ester;
Chloro-{2-oxo-4-[2-oxo-2-(tetrahydrofuran-2-yl)-ethylsulfanyl]-3-phenylacetylamino-azetidin-1-yl}-acetic acid 4-nitro-benzyl ester;
Chloro-{2-oxo-4-[2-oxo-2-(tetrahydrofuran-2-yl)-ethylsulfanyl]-3-phenylacetylamino-azetidin-1-yl}-acetic acid allyl ester;
Hydroxy-{2-oxo-4-[2-oxo-2-(tetrahydrofuran-2-yl)-ethylsulfanyl]-3-phenylacetylamino-azetidin-1-yl}-acetic acid 4-nitro-benzyl ester;
Hydroxy-(2-oxo-4-[2-oxo-2-(tetrahydrofuran-2-yl)-ethylsulfanyl]-3-phenylacetylamino-azetidin-1-yl}-acetic acid allyl ester;

and salts thereof.

Compounds of formula (V) include:
Hydroxy-(2-mercapto-4-oxo-3-phenylacetylamino-azetidin-1-yl)-acetic acid 4-nitro-benzyl ester;
Hydroxy-(2-mercapto-4-oxo-3-phenylacetylamino-azetidin-1-yl)-acetic acid allyl ester;

and salts thereof.

Compounds of formula **(VI)** include:
(3-Benzyl-7-oxo-4-thia-2,6-diaza-bicyclo[3.2.0]hept-2-en-6-yl)-hydroxy-acetic acid 4-nitro-benzyl ester;
(3-Benzyl-7-oxo-4-thia-2,6-diaza-bicyclo[3.2.Ojhept-2-en-6-yl)-hydroxy-acetic acid allyl ester;
(3-Benzyl-7-oxo-4-thia-2,6-diaza-bicyclo[3.2.0]hept-2-en-6-yl)-oxo-acetic acid 4-nitro-benzyl ester;
(3-Benzyl-7-oxo-4-thia-2,6-diaza-bicyclo[3.2.0]hept-2-en-6-yl)-oxo-acetic acid allyl ester;

and salts thereof.

The foregoing novel compounds are useful in the preparation of 3-cyclic-ether-substituted cephalosporins.

### DETAILED DESCRIPTION OF THE INVENTION

The process of the present invention and the preparation of the compounds of the present invention are illustrated in the following reaction schemes. Except where otherwise indicated, in the reaction schemes and discussion that follow, substituents wherein R¹, R², R³, R⁴, X, X', L₁, L₂ and L₃ are as defined above.

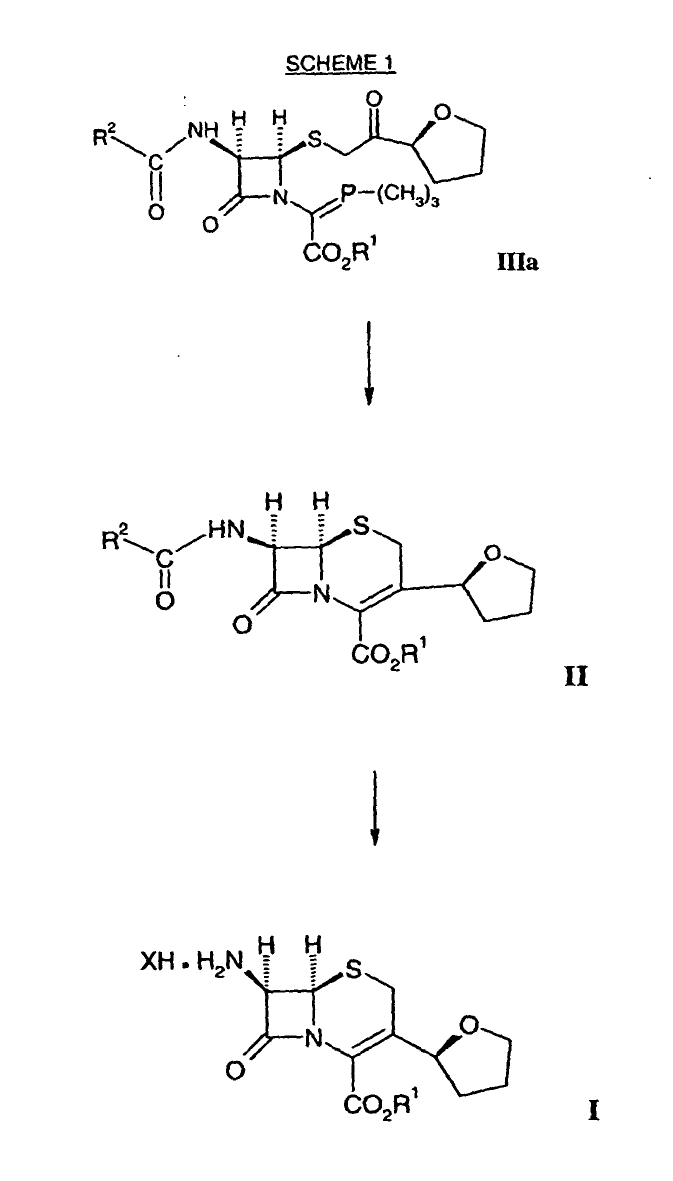

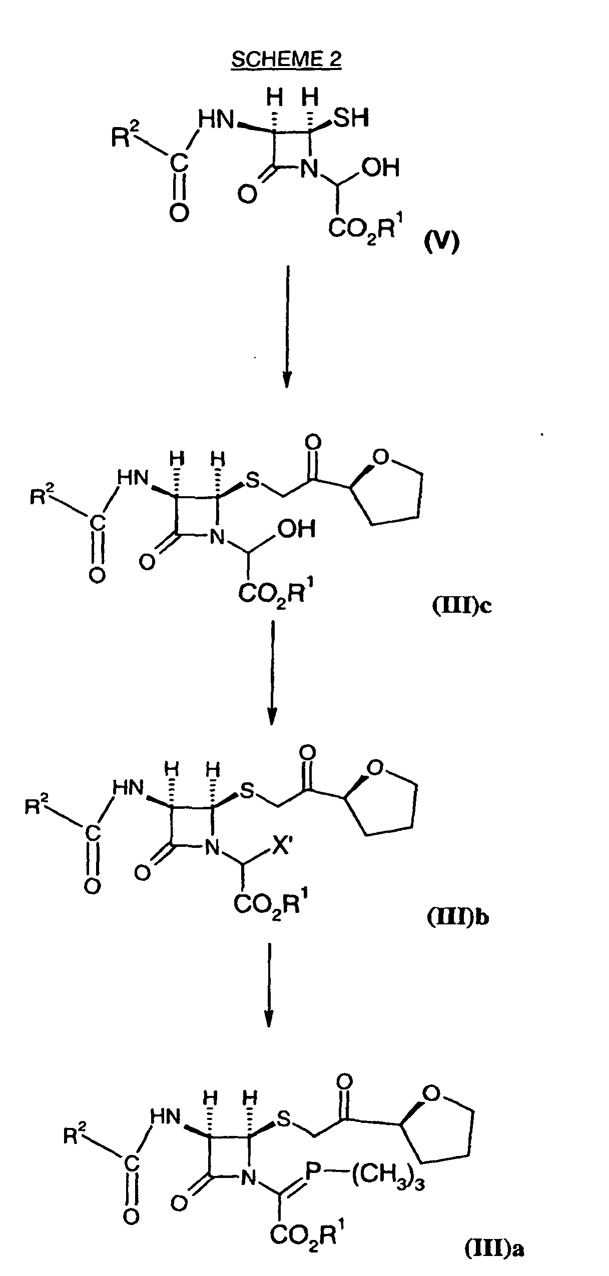

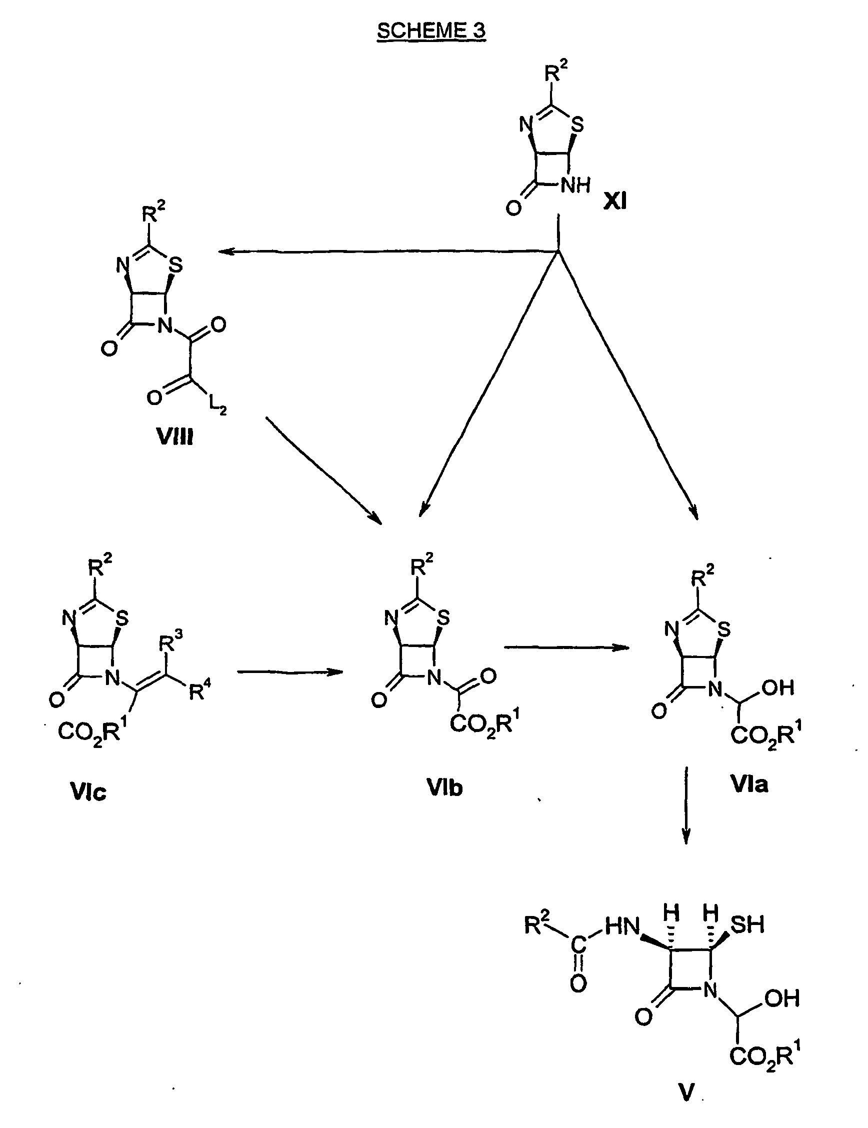

Scheme 1 refers to the preparation of a compound of formula **(I).** Referring to Scheme 1, a compound of formula **(I)** wherein R¹ is preferably *para*-nitrobenzyl can be prepared by reaction of a compound of formula **(II)** wherein R¹ is preferably para-nitrobenzyl, and R² is preferably C₆₋₁₀arylC₁₋₆alkyl, such as benzyl, with an acid in a solvent. Suitable acids include Lewis Acids, such as phosphorus pentachloride or phosphorus pentabromide, preferably phosphorus pentachloride. Suitable solvents include toluene, xylene, tetrahydrofuran, methylene chloride or acetonitrile; preferably methylene chloride. The aforesaid process can be conducted at a temperature of about -40°C to about +40°C. The aforesaid process is conducted for a period of from about 1 hour to about 24 hours.

A compound of formula **(II)** wherein R¹ is preferably *para-*nitrobenzyl, and R² is preferably C₆₋₁₀arylC₁₋₆alkyl, such as benzyl, can be prepared by cyclizing a compound of formula **(IIIa)**, wherein R¹ is preferably para-nitrobenzyl; and R² is preferably C₆₋₁₀arylC₁₋₆alkyl, such as benzyl; by heating said compound of formula **(IIIa)** in a solvent.

The aforesaid process for the conversion of compounds of formula **(IIIa)** into compounds of formula **(II)** is a so called intramolecular Wittig-type reaction and is typically conducted by heating the above compound of formula **(IIIa)**. Suitable solvents include toluene, xylene, tetrahydrofuran, methylene chloride and acetonitrile, preferably methylene chloride. The aforesaid process is conducted at a temperature of from about 40°C to about 160°C. The aforesaid process is conducted for a period of from about 1 hour to about 24 hours, preferably about 16 hours.

The aforesaid conversion of the compound of formula **(IIIa)** to the compound of formula **(I)** can be performed as a two step process in which the compound of formula **(II)** may be isolated but is preferably carried out as a one step reaction without isolation of the phosphorus ylide.

Compounds of formula **(IIIa)** can be prepared by the methods of Scheme 2.

Scheme 2 refers to the preparation of compounds of the formula **(IIIa),** wherein R¹ is preferably *para-*nitrobenzyl; and R² is preferably C₆₋₁₀arylC₁₋₈alkyl, such as benzyl; by the processes of the present invention. Compounds of the formula **(IIIa)** are intermediates useful in the preparation of compounds of formula **(I)** in Scheme 1.

Referring to Scheme 2, the aforesaid compound of formula **(IIIa)** can be prepared by reacting a compound of formula **(IIIb),** wherein R¹ is preferably para-nitrobenzyl; and R² is preferably C₆₋₁₀arylC₁₋₆alkyl, such as benzyl; and X is preferably chloro, with trimethylphoshine, in a solvent, optionally in the presence of a suitable base.

Suitable solvents include tetrahydrofuran, acetonitrile and methylene chloride, preferably tetrahydrofuran. Suitable bases include imidazole, 2,6-lutidine, pyridine, N-methylmorpholine or sodium bicarbonate, preferably sodium bicarbonate. Preferably the reaction is conducted with the suitable base during work up. The aforesaid process is conducted at a temperature of from about -40°C to about -20°C. The aforesaid process is conducted for a period of from about 30 minutes to about 1 hour.

A compound of formula **(IIIb),** wherein R¹ is preferably *para*-nitrobenzyl; and R² is preferably C₆₋₁₀arylC₁₋₆alkyl, such as benzyl; can be prepared by reacting a compound of formula **(IIIc),** wherein R¹ is preferably *para*-nitrobenzyl; and R² is preferably C₆₋₁₀arylC₁₋₆alkyl, such as benzyl; with a halogenating agent in the presence of a base in a solvent. Suitable halogenating agents include thionyl chloride, thionyl bromide, phosphorus tribromide or phosphorus trichloride, preferably thionyl chloride. Suitable bases include pyridine, 2,6-lutidine, N-methylmorpholine or imidazole, preferably 2,6-lutidine. Suitable solvents include tetrahydrofuran or methylene chloride, preferably methylene chloride. The aforesaid process is conducted at a temperature of from about -40°C to about -20°C, preferably about -20°C. The aforesaid process is conducted for a period of from about 15 minutes to about 1 hour, preferably about 1 hour.

A compound of formula **(IIIc)**, wherein R¹ is preferably para-nitrobenzyl; and R² is preferably C₆₋₁₀arylC₁₋₆alkyl, such as benzyl; can be prepared by reacting a compound of formula **(V),** wherein R¹ is preferably para-nitrobenzyl; and R² is preferably C₆₋₁₀arylC₁₋₆alkyl, such as benzyl; with a compound of formula **(IV)** 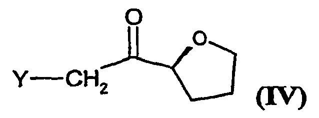
wherein Y is a leaving group such as bromo, chloro, fluoro, iodo or tosylate, preferably bromo, in a solvent. Suitable solvents include alcohol, such as methanol, ethanol and propanol; methylene chloride; acetone; dimethylformamide; or mixtures thereof. The aforesaid process is conducted at a temperature of from about 10°C to about 25°C. The aforesaid process is conducted for a period of from about 4 hours to about 24 hours.

Compounds of formula **(IV)** are known compounds and can be prepared by standard methodology. For example, compounds of formula **(IV),** in which Y is chloro or bromo, can be prepared from a compound of formula **(IVa)** 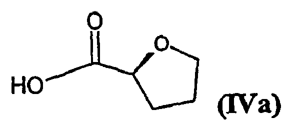
by reacting said compound of formula **(IVa)** with a halogenating agent, such as thionyl chloride or phophorus tribromide, to form the corresponding acid halide (such as chloroformyltetrahydrofuran or bromoformyltetrahydrofuran). Said acid halide is reacted with diazomethane to form a diazo compound. The resulting diazo compound is then treated with hydrogen chloride or hydrogen bromide to form the corresponding compound of formula **(IV).**

Compounds of formula **(IVa),** the corresponding acid halides and diazomethane are commercially available.

Alternatively, the compound of formula **(IV)** can be prepared *in situ* by reacting the corresponding carboxylic acid of formula **(IVb)** 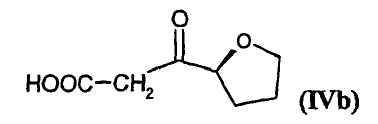
with a halogenating agent in methanol or water solution; and subsequently exposing the solution to an acid, preferably para-toluene sulfonic acid. Suitable halogenating agents include bromine, chlorine or iodine, preferably bromine.

Those skilled in the art would understand that in the process of the invention, the compound of formula (IV) made *in situ* is then reacted with compounds of formula (V) to prepare compounds of formula (IIIc), by the method described above.

Compounds of the formula (V) can be prepared by the methods of Scheme 3.

Scheme 3 refers to the preparation of compounds of the formula (V), wherein R¹ is preferably *para*-nitrobenzyl; and R² is preferably C₆₋₁₀arylC₁₋₆alkyl, such as benzyl; by the processes of the present invention. Compounds of the formula (V) are useful intermediates in the preparation of compounds of formula (I), via compounds of the formula **(IIIa)**. The conversion of compounds of formula (V) into compounds of formula I are described in Schemes 1 and 2. Referring to Scheme 3, a compound of formula (V) can be prepared by reacting a compound of formula (VIa), wherein R¹ is preferably para-nitrobenzyl; and R² is preferably C₆₋₁₀arylC₁₋₆alkyl, such as benzyl; with an acid in a solvent. Suitable acids include para-toluene sulfonic acid and methane sulfonic acid, preferably para-toluene sulfonic acid. Suitable solvents include methylene chloride, tetrahydrofuran, acetone or mixtures thereof, preferably methylene chloride. The aforesaid process is conducted at a temperature of from about 20°C to about 25°C. The aforesaid process is conducted for a period of from about 2 hours to about 24 hours.

A compound of formula **(VIa)**, wherein R¹ is preferably para-nitrobenzyl; and R² is preferably C₆₋₁₀arylC₁₋₆alkyl, such as benzyl; can be prepared by reacting a compound of formula **(VIb)**, wherein R¹ is preferably *para*-nitrobenzyl; and R² is preferably

C₆₋₁₀arylC₁₋₆alkyl, such as benzyl; with a reducing agent; in a solvent. Suitable reducing agents include sodium borohydride, sodium cyanoborohydride, borane and sodium triacetoxy borohydride, preferably sodium triacetoxyborohydride or sodium borohydride. Suitable solvents include acetic acid, methylene chloride, tetrahydrofuran, alcohol (such as isopropanol) or mixtures thereof. When the reducing agent is sodium triacetoxy borohydride, preferably the solvent is methylene chloride. When the reducing agent is sodium borohydride, preferably the solvent is acetic acid. The aforesaid process is conducted at a temperature of from about 20°C to about 66°C. The aforesaid process is conducted for a period of from about 4 hours to about 24 hours.

Alternatively, the compound of formula **(VIa),** wherein R¹ is preferably para-nitrobenzyl; and R² is preferably C₆₋₁₀arylC₁₋₆alkyl, such as benzyl; can be prepared by reacting a compound of formula **(XI)**, wherein R² is preferably C₆₋₁₀arylC₁₋₆alkyl, such as benzyl, with a compound of formula **(X),** 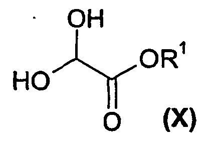
wherein R¹ is preferably para-nitrobenzyl, in the presence of a base in a solvent. Suitable bases include diisopropylamine, triethylamine, pyridine and 2,6-lutidine; preferably triethylamine; more preferably the triethylamine is catalytic. Suitable solvents include methylene chloride, tetrahydrofuran or mixtures thereof. The aforesaid process is conducted at a temperature of from about 20°C to about 25°C. The aforesaid process is conducted for a period of from about 30 minutes to about 2 hours, preferably about 1 hour.

Compounds of formulae **(X)** and **(XI)** are individually known and are commercially available.

A compound of formula (VIb), wherein R¹ is preferably para-nitrobenzyl; R² is preferably C₆₋₁₀arylC₁₋₆alkyl, such as benzyl; can be prepared by reacting a compound of formula **(VIII)**, wherein R² is preferably C₆₋₁₀arylC₁₋₆alkyl, such as benzyl, and said L₂ is halo, such as bromo or chloro, with a compound of formula **(VII)**

R¹-OH (VII)

wherein R¹ is preferably para-nitrobenzyl; in a solvent, in the presence of a base.

Said compound of formula **(VIII)** is prepared by reacting said compound of formula **(XI)** with a compound of formula **(IX)** 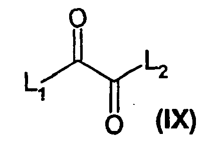
wherein each of L₁ and L₂ is a leaving group, such as halo, preferably chloro, in a solvent, optionally in the presence of a base. Suitable solvents include methylene chloride, tetrahydrofuran, or mixtures thereof, preferably methylene chloride. Suitable bases include diisopropylamine, triethylamine, pyridine and 2,6-lutidine, preferably triethylamine. The aforesaid process is conducted at a temperature of about -78°C to about 25°C, preferably about -78°C. The aforesaid process is conducted for a period of from about 5 minutes to about 10 minutes, preferably about 5 minutes.

The compound of formula **(VIII)** may be isolated, or may be carried on to the next step without isolation. Preferably the compound of formula **(VIII)** is isolated.

Compounds of formula **(VII)** and **(IX)** are commercially available.

Alternatively, a compound of formula **(VIb)**, wherein R¹ is preferably *para*-nitrobenzyl; and R² is preferably C₆₋₁₀arylC₁₋₆alkyl, such as benzyl; can be prepared by reacting a compound of formula (VIc), wherein R¹ is preferably para-nitrobenzyl; R² is preferably C₆₋₁₀arylC₁₋₆alkyl, such as benzyl; R³ is preferably C₁₋₆alkyl, such as methyl; and R⁴ is preferably C₁₋₆alkyl, such as methyl; with an oxidizing agent, in a solvent. Suitable oxidizing agents include ozone. Suitable solvents include methylene chloride, tetrahydrofuran or mixtures thereof, preferably methylene chloride. The aforesaid process is conducted, at a temperature of about -70°C. The aforesaid process is conducted for a period of from about 1 hour to about 24 hours.

A compound of formula **(VIc)** is commercially available.

Alternatively, a compound of formula **(VIb),** wherein R¹ is preferably *para*-nitrobenzyl, and R² is preferably C₆₋₁₀arylC₁₋₆alkyl, such as benzyl; can be prepared by reacting a compound of formula **(XI),** wherein R² is preferably C₆₋₁₀arylC₁₋₆alkyl, such as benzyl; with a compound of formula **(XII)** 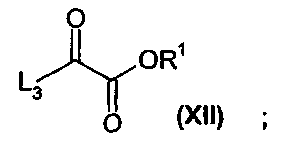
wherein R¹ is preferably para-nitrobenzyl, and L₃ is a leaving group, such as halo, preferably chloro, in a solvent in the presence of a base. Suitable solvents include methylene chloride, tetrahydrofuran or mixtures thereof. Suitable bases include diisopropylamine, triethylamine, pyridine or 2,6-lutidine. The aforesaid process is conducted at a temperature of from about-40°C to about 25°C. The aforesaid process is conducted for a period of about 5 minutes to 15 minutes.

Compounds of formula **(XII)** are commercially available.

The compounds of formula **(I)** are useful for the preparation of a 3-cyclic-ether-substituted cephalosporin, i.e., the active compound, of formula **(Ia)** 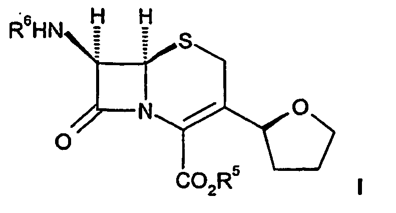
wherein
the group CO₂R⁵ is a carboxylic acid or a carboxylate salt; and
R⁶ has a formula: 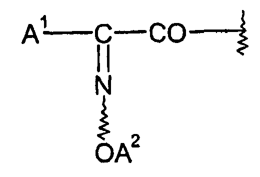
wherein
A¹ is C₆₋₁₀aryl, C₁₋₁₀heteroaryl or C₁₋₁₀heterocyclyl;
A² is hydrogen, C₁₋₆alkyl, C₃₋₁₀cycloalkyl, C₆₋₁₀aryl, C₁₋₆alkyl(CO)(C₁₋₆)alkyl-O-, HO(CO)(C₁₋₆)alkyl, mono-(C₆₋₁₀aryl)(C₁₋₆alkyl), di-(C₆₋₁₀aryl)(C₁₋₆alkyl) or tri-(C₆₋₁₀aryl)(C₁₋₆alkyl).

The process for converting the aforesaid compound of formula (I) into the aforesaid compound of formula (Ia) is referred to in WO-A-02 46 198 entitled "Coupling Process And Intermediates Useful For Preparing Cephalosporins". The active compound possesses activities against gram positive and gram negative bacteria. Methods for assaying the activity and methods for formulating and administering the active compounds are disclosed in United States Patent No. 6,020,329, issued February 1, 2000. Methods of treatments are also described in the aforesaid patent, hereby incorporated by reference.

The compounds prepared by the process of this invention can be crystallized or recrystallized from solvents such as organic solvents. In such cases solvates can be formed. This invention includes within its scope stoichiometric solvates including hydrates as well as compounds containing variable amounts of water that can be produced by processes such as lyophilization.

The following Examples illustrate the preparation of the compounds of the present invention. Melting points are uncorrected. NMR data are reported in parts per million (ppm) and are referenced to the deuterium lock signal from the sample solvent (deuteriochloroform unless otherwise specified). Commercial reagents were utilized without further purification. Room or ambient temperature refers to 20°C to 25°C. All non-aqueous reactions were run under a nitrogen atmosphere for convenience and to maximize yields. Concentration at reduced pressure means that a rotary evaporator was used. TLC stands for thin liquid chromatography. HPLC stands for high pressure liquid chromatography. GC stands for gas chromatography. CAM stands for ceric ammonium molybdate. UV stands for ultra violet.

### Example 1

### 7-Amino-8-oxo-3-(tetrahydrofuran-2-yl)-5-thia-1-aza-bicyclo[4.2.0]oct-2-ene-2-carboxylic acid 4-nitro-benzyl ester, hydrochloride salt

Thionyl chloride (45 ml, 0.615 mol) was added dropwise to a solution of hydroxy-{2-oxo-4-[2-oxo-2-(tetrahydrofuran-2-yl)-ethylsulfanyl]-3-phenylacetylamino-azetidin-1-yl}-acetic acid 4-nitro-benzyl ester (202 g, 0.362 mol) and 2,6-lutidine (58 ml, 0.500 mol) in dichloromethane (4 liters) at -20°C. After stirring for 1 hour, the solution was washed twice with saturated sodium chloride (1 liter) and concentrated to form chloro-{2-oxo-4-[2-oxo-2-(tetrahydrofuran-2-yl)-ethylsulfanyl]-3-phenylacetyl amino-azetidin-1-yl}-acetic acid 4-nitro-benzyl ester, which was carried on to the next step without isolation. To the concentrated solution was added trimethylphosphine in tetrahydrofuran solution (110 ml, 3M, 330 mmol), the solution stirred for 1 hour, washed with diluted sodium hydrogen carbonate and saturated sodium chloride to form {2-Oxo-4-[2-oxo-2-(tetrahydrofuran-2-yl)-ethylsulfanyl]-3-phenylacetylamino-azetidin-1-yl}-(trimethyl-α-phosphanylidene)-acetic acid 4-nitro-benzyl ester, which was carried on to the next step without isolation. After stirring at reflux for 16 hours, the solution was washed with water and saturated sodium chloride to form 8-Oxo-7-phenylacetylamino-3-(tetrahydrofuran-2-yl)-5-thia-1-aza-bicyclo[4.2.0] oct-2-ene-2-carboxylic acid 4-nitro-benzyl ester, which was carried on to the next step without isolation. The solution was concentrated and cooled to -40°C followed by a dropwise addition of phosphorus pentachloride (104 g, 0.5 mol). α-Picoline (92 ml) in dichloromethane (60 ml) solution was added while maintaining the temperature between -40°C to -30°C. The mixture was stirred for 1 hour followed by the addition of isopropanol (660 ml). The reaction mixture was warmed to 22°C, granulated, filtered and dried to give the title compound (250 g, 45%).

### Example 2

### 8-Oxo-7-phenylacetylamino-3-(tetrahydrofuran-2-yl)-5-thia-1-aza-bicyclo[4.2.0] oct-2-ene-2-carboxylic acid 4-nitro-benzyl ester

The title compound was prepared in Example 1 but was carried on to the next step without isolation.

### Example 3

### {2-Oxo-4-[2-oxo-2-(tetrahvdrofuran-2-yl)-ethylsulfanyl]-3-phenylacetylamino-azetidin-1-yl}-(trimethyl-α-phosphanylidene)-acetic acid 4-nitro-benzvl ester

The title compound was prepared in Example 1 but was carried on to the next step without isolation.

### Example 4

### Chloro-{2-oxo-4-[2-oxo-2-tetrahydrofuran-2-yl)-ethylsulfanyl]-3-phenylacetyl aminoazetidin-1-yl}-acetic acid 4-nitro-benzyl ester

The title compound was prepared in Example 1 but was carried on to the next step without isolation.

### Example 5

### Hydroxy-{2-oxo-4-[2-oxo-2-tetrahydrofuran-2-yl)-ethylsulfanyl]-3-phenylacetyl aminoazetidin-1-yl}-acetic acid 4-nitro-benzyl ester, hydrochloride salt

Bromine (51 g) and methanol (270 mL) were combined followed by a dropwise addition of a (S)-1-(tetrahydro-2-furanyl)-ethanone (30 g) in methanol (30 mL) solution at 30°C. An aqueous sodium thiosulfate solution was then added followed by methylene chloride (300 mL). The layers were separated and the organic layer washed twice with an aqueous solution of sodium bicarbonate (300 mL). The resulting organic layer was concentrated followed by the addition of acetone (600 mL) and para-toluene sulfonic acid (6 g). After heating to reflux for 2 hours, the reaction was cooled and (3-benzyl-7-oxo-4-thia-2,6-diaza-bicyclo[3.2.0]hept-2-en-6-yl)-hydroxy-acetic acid 4-nitro-benzyl ester (100 g) and an additional para-toluene sulfonic acid (6 g) were charged. Hydroxy-(2-mercapto-4-oxo-3-phenylacetylamino-azetidin-1-yl)-acetic acid 4-nitro-benzyl ester was formed, and was carried on to the next step without isolation. The resulting solution was stirred for 2 hours followed by a pH adjustment between 3 to 4 by using pyridine. The reaction was concentrated followed by the addition of water (180 mL), methylene chloride (600 mL) and hydrochloric acid (9 mL, 15%) to adjust the pH between 1 and 2. The layers were separated and the methylene chloride displaced with methanol (600 mL). Isopropanol (300 mL) was added to complete the precipitation and the resulting slurry was granulated, filtered and the cake washed with isopropanol. The product was dried under vacuo to give the title compound.

### Example 6

### Hydroxy-(2-mercapto-4-oxo-3-phenylacetylamino-azetidin-1-yl)-acetic acid 4-nitro-benzyl ester

The title compound was prepared in Example 5 but was carried on to the next step without isolation.

### Example 7

### (3-Benzyl-7-oxo-4-thia-2,6-diaza-bicyclo[3.2.0]hept-2-en-6-yl)-oxo-acetic acid 4-nitro-benzyl ester, hydrochloride salt

### METHOD A:

To a magnetically stirred, nitrogen blanketed, 250 ml round flask was added: 5.0 g (22.9 mmol, 1.0 eq.) 3-benzyl-4-thia-2,6-diaza-bicyclo[3.2.0]hept-2-en-7-one, 5.98 g (26.3 mmol, 1.15 eq.) para-nitrobenzyl glyoxalate monohydrate and 75 ml methylene chloride. To the stirring slurry was added 0.22 ml (1.6 mmol, 0.7 eq.) triethylamine. Solids will slowly go into solution after addition of triethylamine. Stir for approximately 1 hour. Typically, all solids will be in solution and ethyl acetate (ethyl acetate, CAM Stain) shows no remaining 3-benzyl-4-thia-2,6-diaza-bicyclo[3.2.0]hept-2-en-7-one.

Acidify the solution to pH 4 to 5 with 0.1 M hydrochloric acid. Settle and separate the layers. Lower (organic) layer is washed twice with 50 ml water (brine may be added for persistent emulsions). The solution is dried with anhydrous magnesium sulfate and concentrated under vacuum. 9.37 g oily foam, 96% yield of the title compound.

### METHOD B:

Isopropanol (500 mL), methylene chloride (1800 mL) and (1R)-(4-nitrophenyl)methyl ester-α,1-methylethylidene)-7-oxo-3-(phenylmethyl)-4-thia-2,6-diazabicyclo[3.2.0]hept-2-ene-6-acetic acid (250 g) were combined and the reaction mixture cooled at -70°C. To the cooled reaction mixture, ozone was bubbled until the ozonolysis was completed to form 3-benzyl-7-oxo-4-thia-2,6-diaza-bicyclo[3.2.0]hept-2-en-6-yl)-hydroxy-acetic acid 4-nitro-benzyl ester, which was carried on to the next step without isolation. To the resulting solution, a mixture of glacial acetic acid (625 mL) and isopropanol (750 mL) was added followed by a mixture of isopropanol (100 mL), water (100 mL) and sodium borohydride (22 g). After the reduction was completed, a sodium metabisulfite in water solution was added followed by the pH adjustment to 1.5 to 2.5 with hydrochloric acid (15%). The layers were separated and the organic layer was washed twice with aqueous sodium chloride (1000 mL). The organic layer was concentrated under vacuum and the resulting slurry granulated, filtered, and the cake washed with isopropanol. The product was dried under vacuo to give the title compound.

### Example 8

### (3-Benzyl-7-oxo-4-thia-2,6-diaza-bicyclo[3.2.0]hept-2-en-6-yl)-3-methyl-but-2-enoic acid 4-nitro-benzyl ester

### METHOD A:

To a round bottom flask equipped with a magnetic stirrer, which was placed under nitrogen atmosphere, was added 3-benzyl-4-thia-2,6-diaza-bicyclo[3.2.0]hept-2-en-7-one (0.76g, 3.5 mmol, 1.0 equivalents), methylene chloride (8.0 ml) and triethylamine (0.64 ml, 4.6 mmol, 1.3 equivalents). The slurry was cooled to -78° C before addition of a 2M solution of oxalyl chloride (1.85 ml, 3.7 mmol, 1.05 equivalents) in methylene chloride over 1 minute. The color of the solution became a dark red/brown. Thin layer chromatography (ethyl acetate, UV, CAM stain) indicated the reaction was complete after 5 minutes. A solution of (4-Nitrophenyl)methanol (0.54g, 3.5 mmol, 1.0 equivalents) and triethylamine (0.64 ml, 4.6 mmol, 1.3 equivalents) in methylene chloride (5.0 ml) was then added in one portion. Thin layer chromatography (ethyl acetate, UV, CAM stain) indicated the reaction was complete after 5 minutes. The reaction was quenched with water (15 ml). The organic layer was then washed sequentially with saturated aqueous sodium hydrogen carbonate (15 ml) and saturated aqueous sodium chloride (15 ml). After drying with magnesium sulfate and charcoal treatment, the organic solution was concentrated under vacuo to obtain the title compound (1.0g, 2.35 mmol, 67% yield) as a dark brown solid.

### METHOD B:

To a round bottom flask equipped with a magnetic stirrer, which was placed under nitrogen atmosphere, was added 3-benzyl-4-thia-2,6-diaza-bicyclo[3.2.0]hept-2-en-7-one (161 mg, 0.74 mmol, 1.0 equivalent), methylene chloride (10 ml) and triethylamine (0.22 ml, 1.55 mmol., 2.1 eq.). The solution was stirred at 20-25°C and chloro-oxo-acetic acid 4-nitro-benzyl ester (198 mg, 0.81 mmol, 1.1 equivalent) was added in one portion. The initially light yellow solution changed to a light orange color in approximately 10 minutes. The reaction was then washed sequentially with water, saturated aqueous sodium hydrogen carbonate and saturated aqueous sodium chloride. The organic layer was then dried with magnesium sulfate and concentrated under vacuo to obtain the title compound (250 mg 0.55 mmol, 79% yield) as a light orange solid.

### Preparation 1: Chloro-oxo-acetic acid 4-nitro-benzyl ester

To a round bottom flask equipped with a magnetic stirrer, which was placed under nitrogen atmosphere, was added methylene chloride (60 ml), followed by a 2M solution of oxalyl chloride in methylene chloride (15.0 ml, 30 mmol, 1.0 equivalent). The solution was cooled in ice water to 0-5°C. (4-Nitro-phenyl)-methanol (4.59g 30 mmol., 1.0 equivalent) was then added in one portion to the oxalyl chloride solution. After the addition of para-nitrobenzyl alcohol was complete, the reaction was allowed to stir at 20-25°C for 24 hours. The solution was then concentrated under vacuo and titrated with hot hexanes to obtain the title compound (5.6g, 23 mmol, 77% yield) as a white solid.

### Example 9

### 3-Benzyl-7-oxo-4-thia-2,6-diaza-bicyclo[3.2.0]hept-2-en-6-yl)-hydroxy-acetic acid 4-nitro-benzyl ester

The title compound was prepared in Example 8, Method B, but was carried on to the next step without isolation.

### Example 10

### 7-Amino-8-oxo-3-(tetrahydrofuran-2-yl)-5-thia-1-aza-bicyclo[4.2.0]oct-2-ene-2-carboxylic acid allyl ester

To a 10 liter glass vessel was added methylene chloride (4.50 liters) followed by phosphorous pentachloride (277.0 g, 1.33 moles). The vessel was purged with nitrogen and pyridine (350.4 g, 4.43 moles) added at a maximum temperature of 25°C. The solution was then cooled back to -20°C. 8-Oxo-7-phenylacetylamino-3-(tetrahydrofuran-2-yl)-5-thia-1-aza-bicyclo[4.2.0]oct-2-ene-2-carboxylic acid allyl ester (190.0 g, 0.443 moles) was dissolved in methylene chloride (350 ml), added to a header vessel, and charged to the methylene chloride solution at -20°C for approximately 20 minutes. The beaker used for dissolution and the header flask were rinsed with methylene chloride. The solution was allowed to warm to 0°C and stirred at this temperature for one hour.

The solution was then sampled for analysis. Upon completion methanol (3.70 liters) was added at -20°C, while ensuring that the methylene chloride solution did not warm above 10°C. The quenching process typically took 90 minutes after which time the temperature was allowed to rise to 0°C and the solution was then stirred for 30 minutes. A 7% sodium carbonate solution (10 liters) was added to the methanol solution at a maximum temperature of 5°C bringing the pH to 7 to 7.5. Some foaming was observed. The solution was then transferred to a 20-liter separating funnel and the two phases separated. The aqueous phase was then extracted with methylene chloride (1.5 liters). Afterwards, the combined methylene chloride phases were washed with 20% of saturated sodium chloride (1.5 kg) and dried over sodium sulphate (50 g) to give the title compound.

### Example 11

### 8-Oxo-7-phenylacetylamino-3-(tetrahydrofuran-2-yl)-5-thia-1-aza-bicyclo[4.2.0]oct-2-ene-2-carboxylic acid allyl ester

To a 100-liter glass vessel was added toluene (47 liters) and {2-Oxo-4-[2-oxo-2-(tetrahydrofuran-2-yl)-ethylsulfanyl]-3-phenylacetylamino-azetidin-1-yl}-(trimethyl-α-phosphanylidene)-acetic acid allyl ester (1990 g). The solution was purged with nitrogen and brought to reflux. Any water present was collected and the solution was refluxed for 20 hours. After sampling for TLC/HPLC analysis, the solution was cooled back to ambient temperature. The solution was then run through Silica Gel 60 (4.5 kg), with the silica being further eluted with additional toluene (33 liters). The toluene was then stripped under vacuo at a maximum temperature of 60°C. Ethyl acetate was then added and was then stripped under vacuo at a maximum temperature of 60°C. To the semi solid oil was added tert-butyl methyl ether (2.5 liters) and the solution stirred overnight. The crystalline product was filtered off and washed with further tert-butyl methyl ether (0.3 liters). The mother liquors were concentrated and resubjected to silica chromatography (dissolved in 5 liters of toluene, added onto silica, eluted with 15 liters of toluene) and crystallized in the same fashion to afford a second crop. The product was isolated as a white crystalline solid. Yields range from 70% to 80%.

### Example 12

### {2-Oxo-4-[2-oxo-2-(tetrahydrofuran-2-yl)-ethylsulfanyl]-3-phenylacetylamino-azetidin-1-yl}-(trimethyl-α-phosphanylidene)-acetic acid allyl ester

The solution of hydroxy-{2-oxo-4-[2-oxo-2-(tetrahydrofuran-2-yl)-ethylsulfanyl]-3-phenylacetylamino-azetidin-1-yl}-acetic acid allyl ester in tetrahydrofuran, which was obtained from example 14, was further diluted with additional tetrahydrofuran (total tetrahydrofuran was 12 liters). The solution was cooled back to -20°C under nitrogen and 2,6-lutidene (654.0g, 6.09 moles) was added, followed by a dropwise addition of thionyl chloride (724.0g, 6.09 moles) at a maximum temperature of -20°C. After a thirty minute stirring, the solution was allowed to warm to -10°C and sampled for TLC. The TLC showed that the starting material was converted into chloro-{2-oxo-4-[2-oxo-2-(tetrahydrofuran-2-yl)-ethylsulfanyl]-3-phenylacetylamino-azetidin-1-yl}-acetic acid allyl ester to completion. The precipitated compounds were then filtered off and washed further with tetrahydrofuran. The tetrahydrofuran solution was then concentrated under vacuo at a maximum temperature of 30°C, redissolved in fresh tetrahydrofuran (6 liters) and cooled back to -10°C. After stirring overnight at ambient temperature, the solution was sampled for completion, diluted with ethyl acetate (35 liters) and washed with 5% sodium bicarbonate (20 liters) and 20% saturated sodium chloride (20 liters). The ethyl acetate was then stripped under vacuo at a maximum temperature of 40°C to afford thick dark oil. The yields range from 88% to 90%.

### Example 13

### Chloro-{2-oxo-4-[2-oxo-2-(tetrahydrofuran-2-yl)-ethylsulfanyl]-3-phenylacetylamino-azetidin-1-yl}-acetic acid allyl ester

The title compound was prepared in Example 12, but was carried on to the next step without isolation.

### Example 14

### Hydroxy-{2-oxo-4-[2-oxo-2-(tetrahydrofuran-2-yl)-ethylsulfanyl]-3-phenylacetylamino-azetidin-1-yl}-acetic acid allyl ester

To a 20-liter flask was added methylene chloride (10.0 liters), tetrahydrofuran (1.0 liter) and (3-benzyl-7-oxo-4-thia-2,6-diaza-bicyclo[3.2.0]hept-2-en-6-yl)-hydroxy-acetic acid allyl ester (2016 g, 6.05 moles), which was obtained from Example 15. To this solution was added 45% aqueous para-toluene sulphonic acid solution (500.0 g). After a three hour stirring the solution was sampled for completion with TLC. The solution was then transferred to a 50 liter glass separating vessel, and methylene chloride was added (5 liters) followed by water (2 liters). The separated organic phase was then washed with water (4 liters). The methylene chloride phase was then dried over sodium sulphate to afford a dry solution of hydroxy-(2-mercapto-4-oxo-3-phenylacetylamino-azetidin-1-yl)-acetic acid allyl ester in methylene chloride that was then used without delay. To the above solution was added 86% of the solution of 2-bromoacetyltetrahydrofuran in methylene chloride (6.3 moles). The resultant solution was stripped under vacuo at a maximum temperature of 30°C to 50% of its volume. Pyridine (503.1 g, 6.36 moles) was added at a maximum temperature of 10°C. The solution was stirred overnight, diluted with methylene chloride (10 liters) and washed twice with water (10 liters total) then once with saturated sodium chloride (10%, 10 liter). After drying over sodium sulphate, the solution was concentrated under vacuo at a maximum temperature of 40°C to ensure dryness. The solution was redissolved In tetrahydrofuran (5 liter) for use in the next step. If storage was required, the tetrahydrofuran solution was stored and dried before use.

### Preparation 1: 2-bromoacetyltetrahydrofuran

To a 20-liter glass vessel was added methylene chloride (10.0 liters) followed by acetyltetrahydrofuran (838.0 g, 7.34 moles). The solution was then cooled back to -10°C and triethylamine was added (854.0g. 8.44 moles). The vessel was purged with nitrogen and trimethylsilane triflate (1713.0 g, 7.71 moles) was added dropwise at a maximum temperature of -8°C. Addition was typically complete in 45 minutes. After 15 minutes stirring, a sample was removed for TLC and GC analysis, which showed that the reaction was completed. N-bromosuccinimide (1340g, 7.53 moles) was added to the solution at a maximum temperature of -5°C over a period of approximately 45 minutes in six portions. After a 30 minute stirring, the solution was sampled for GC and TLC analysis, which showed that the reaction was completed. The solution was then transferred to a 50-liter separating vessel, and 5% sodium bicarbonate (5 liters) was added with caution. The solution was stirred and separated. The upper aqueous phase was discarded, and the methylene chloride phase was washed with water, dried over sodium sulphate, filtered and stored in a freezer before use in the next step.

### Example 15

### (3-Benzyl-7-oxo-4-thia-2,6-diaza-bicyclo[3.2.0]hept-2-en-6-yl)-hydroxy-acetic acid allyl ester

To a 50-liter glass vessel was added methylene chloride (20.6 liters) followed by 3-benzyl-4-thia-2,6-diazabicyclo[3.2.0]hept-2-en-7-one (1700 g, 7.79 moles). To this suspension was added allyl glyoxylate monohydrate (1285 g, 9.74 moles) followed by sufficient triethylamine (about 175 g) to bring the pH of the solution to 7.5-7.9. After a 1 hour stirring, the solution was sampled for TLC/HPLC analysis. Upon completion, the solution was quenched with 0.1 M of hydrochloric acid (2.75 liters) to a pH of 4.50-5.00. The upper aqueous phase was discarded, and the methylene chloride phase was washed with water (8 liters) and saturated sodium chloride (8 liters). The solution was dried over sodium sulphate and concentrated to thick oil. The oil was dispersed in hexane (5 liters), filtered, and reslurried in tert-butyl methyl ether (5 liters) before filtration and washing with further tert-butyl methyl ether. Air drying afforded an off white crystalline product. Yields range from 72-99%.

While the invention has been described and illustrated with reference to certain particular embodiments thereof, those skilled in the art will appreciate that various adaptations, changes, modifications, substitutions, deletions, or additions of procedures and protocols may be made without departing from the spirit and scope of the invention. It is intended, therefore, that the invention be defined by the scope of the claims that follow and that such claims be interpreted as broadly as is reasonable.

## Claims

1. A process for preparing a compound of formula **(I):** 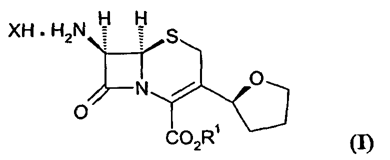
wherein R¹ is para-nitrobenzyl or allyl and X is halo, comprising the steps of:
(a) reacting a compound of formula **(IIIc)** 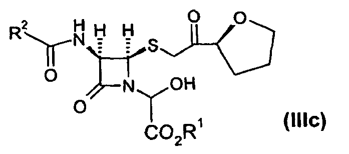
wherein said R¹ is para-nitrobenzyl or allyl and said R² is selected from the group consisting of C₁₋₆alkyl, C₆₋₁₀aryl, C₆₋₁₀arylC₁₋₆alkyl and dithianyl, with a halogenating agent, in a solvent and in the presence of a base;
(b) reacting the resulting compound of formula **(IIIb)** 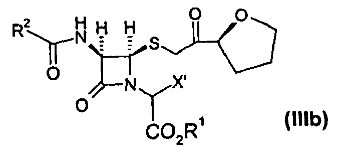
wherein R¹ and R² are described above and X' is halo, with trimethylphosphine, in a solvent and in the presence of a base;
c) cyclizing the resulting trimethylphosphinic compound of formula **(IIIa)** 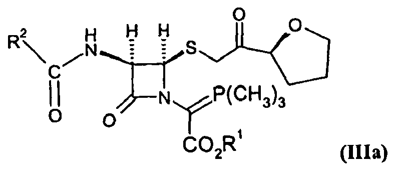
wherein R¹ and R² are described above, in a solvent; and
d) reacting the resulting compound of formula **(II)** 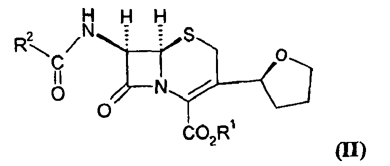
wherein R¹ and R² are described above, with an acid.

2. A compound of formula **(I)** 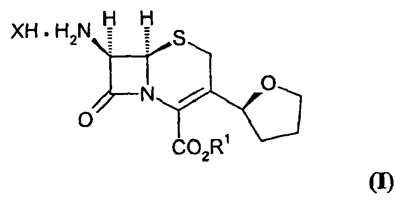
wherein R¹ is para-nitrobenzyl or allyl; and X is halo.

3. A compound according to claim 2, wherein R¹ is para-nitrobenzyl.

4. A compound according to claim 2, wherein R¹ is para-nitrobenzyl and X is chloride.

## Revendications

1. Procédé de préparation d'un composé de formule (I) : 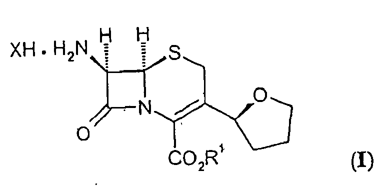
dans laquelle R¹ représente *para*-nitrobenzyle ou allyle, et X représente halogéno, comprenant les étapes de :
(a) réaction d'un composé de formule (IIIc) 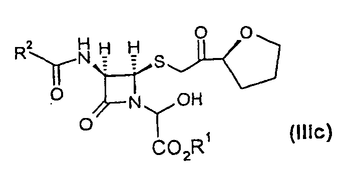
dans laquelle ledit R¹ représente para-nitrobenzyle ou allyle, et ledit R² est sélectionné dans le groupe consistant en alkyle en C₁-C₆, aryle en C₆-C₁₀, (aryle en C₆₋C₁₀) alkyle en C₁-C₆ et dithianyle, avec un agent d'halogénation, dans un solvant et en présence d'une base ;
(b) réaction du composé résultant de formule (IIIb) 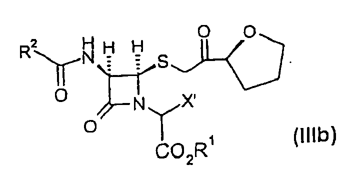
dans laquelle R¹ et R² sont tels que décrits ci-dessus et X¹ représente halogéno, avec de la triméthylphosphine, dans un solvant et en présence d'une base ;
(c) cyclisation du composé triméthylphosphinique résultant de formule (IIIa) 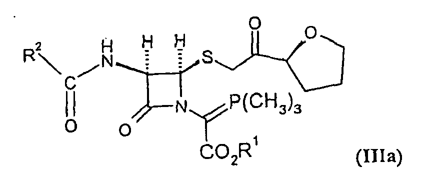
dans laquelle R¹ et R² sont tels que décrits plus haut, dans un solvant ; et
(d) réaction du composé résultant de formule (II) 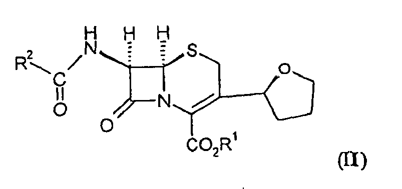
dans laquelle R¹ et R² sont tels que décrits ci-dessus, avec un acide.

2. Composé de formule (I) : 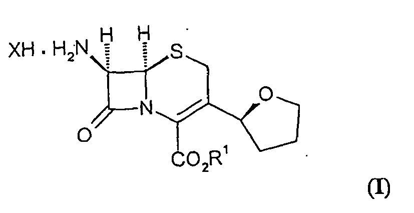
dans laquelle R¹ représente *para*-nitrobenzyle ou allyle ; et X représente halogéno.

3. Composé selon la revendication 2, dans lequel R¹ représente *para*-nitrobenzyle.

4. Composé selon la revendication 2, dans lequel R¹ représente *para*-nitrobenzyle, et X représente chlorure.

## Patentansprüche

1. Verfahren zur Herstellung einer Verbindung der Formel (I): 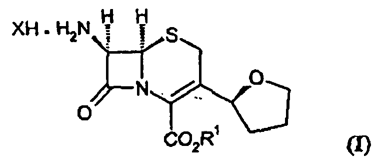
worin R¹ p-Nitrobenzyl oder Allyl bedeutet und X Halogen bedeutet, umfassend die folgenden Stufen:
(a) Umsetzen einer Verbindung der Formel (IIIc) 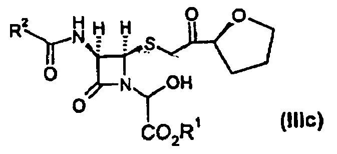
worin R¹ p-Nitrobenzyl oder Allyl bedeutet und R² aus der Gruppe, die aus C₁₋₆-Alkyl, C₆₋₁₀-Aryl, C₆₋₁₀-Aryl-C₁₋₆-alkyl und Dithianyl besteht, ausgewählt ist, mit einem Halogenierungsmittel in einem Lösungsmittel und in Gegenwart einer Base;
(b) Umsetzen der erhaltenen Verbindung der Formel (IIIb) 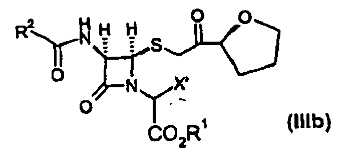
worin R¹ und R² die vorstehend beschriebenen Bedeutungen haben und X' Halogen bedeutet, mit Trimethylphosphin in einem Lösungsmittel und in Gegenwart einer Base;
(c) Cyclisieren der erhaltenen Trimethylphosphin-Verbindung der Formel (IIIa) 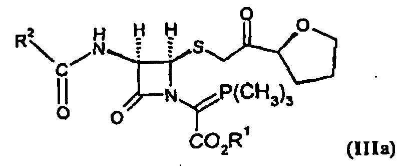
worin R¹ und R² die vorstehend beschriebenen Bedeutungen haben, in einem Lösungsmittel; und
(d) Umsetzen der erhaltenen Verbindung der Formel (II) 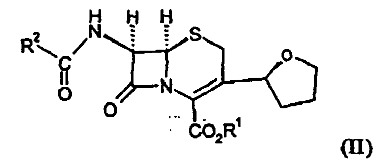
worin R¹ und R² die vorstehend beschriebenen Bedeutungen haben, mit einer Säure.

2. Verbindung der Formel (I) 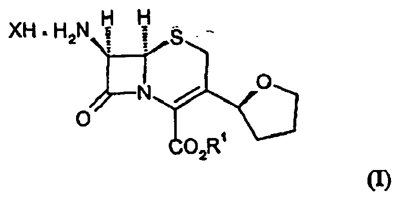
worin R¹ p-Nitrobenzyl oder Allyl bedeutet; und X Halogen bedeutet.

3. Verbindung nach Anspruch 2, wobei R¹ p-Nitrobenzyl bedeutet.

4. Verbindung nach Anspruch 2, wobei R¹ p-Nitrobenzyl bedeutet und X Chlorid bedeutet.
